(19)

Europäisches
Patentamt
European
Patent Office
Office européen
des brevets

(11) **EP 4 627 998 A1**

(12) **EUROPEAN PATENT APPLICATION**

(43) Date of publication:
**08.10.2025 Bulletin 2025/41**

(21) Application number: **24168642.7**

(22) Date of filing: **05.04.2024**

(51) International Patent Classification (IPC):
**A61B 5/00** (2006.01)   **A61B 5/087** (2006.01)
**A61M 16/00** (2006.01)

(52) Cooperative Patent Classification (CPC):
**A61B 5/4818; A61B 5/4815; A61B 5/4836;**
**A61B 5/7275; A61B 5/74; A61M 16/0069;**
**A61M 16/024;** A61B 5/087

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **CERINA, Luca**
**Eindhoven (NL)**

• **DOS SANTOS DA FONSECA, Pedro Miguel**
**Ferreira**
**Eindhoven (NL)**
• **PAPINI, Gabriele**
**5656AG Eindhoven (NL)**
• **VULLINGS, Rik**
**Eindhoven (NL)**
• **OVEREEM, Sebastiaan**
**Eindhoven (NL)**

(74) Representative: **Philips Intellectual Property &**
**Standards**
**High Tech Campus 52**
**5656 AG Eindhoven (NL)**

(54) **COMPUTER PROGRAM PRODUCT, RESPIRATORY SUPPORT DEVICE, AND THERAPY DEVICE**

(57)    There is provided a there is a computer program product, comprising instructions which, when executed by a processing system, cause the processing system to carry out a method for determining a sleep characteristic of a subject during a sleep session. The method comprises receiving sleep stage information and respiratory event information. The method comprises determining a REM value and a NREM value based on the sleep stage information and the respiratory event information. The method comprises determining a first ratio between the REM value and the NREM value; determining whether the first ratio exceeds a first threshold; determining a REM occurrence value representative of an amount of REM sleep during the sleep session; and determining whether the REM occurrence value exceeds a second threshold.

FIG. 4

EP 4 627 998 A1

**Description**

FIELD OF THE INVENTION

The invention relates to a computer program product, comprising instructions which, when executed by a processing system, cause the processing system to carry out a method for determining a sleep characteristic of a subject during a sleep session. Further, the invention relates to a respiratory support device for providing pressurized air to a subject. Further, the invention relates to a therapy device for providing sleep positional therapy.

BACKGROUND OF THE INVENTION

Sleep-disordered breathing (SDB) conditions, such as obstructive sleep apnea (OSA) and central sleep apnea (CSA), are becoming increasingly common, and are particularly prevalent in older people, people with a high body mass index, smokers, heavy drinkers, and people with conditions such as coronary artery disease, hypertension and diabetes mellitus.

SDB conditions are commonly treated using positive airway pressure (PAP) therapy, in which pressurized air is provided to a subject to keep the subject's airways open. When first prescribing PAP therapy, a PAP titration study is carried out for the subject in order to determine a level of airway pressure to be provided to the subject during PAP therapy, as well as a suitable PAP therapy modality (e.g. continuous positive airway pressure, CPAP, bilevel positive airway pressure, BiPAP, or automatic positive airway pressure, APAP) and a suitable subject interface (e.g. a nasal pillow, an oronasal/full-face mask).

During Rapid Eye Movement (REM) sleep, muscle atonia occurs. The muscle atonia increases the collapsibility of the subject's upper airways. Some subjects have substantially more sleep disordered breathing (SDB) events during REM sleep than during non-REM sleep. This type of condition may be referred to as REM-dominant Obstructive Sleep Apnea (REM-dominant OSA, which is further referred to as "REM-OSA"). The PAP therapy is adjusted to reduce the number of SDB events during REM sleep to improve the health of the subject suffering from REM-OSA.

SUMMARY OF THE INVENTION

To reduce the SDB events during REM sleep, a relative high pressure setting of the PAP therapy is needed to keep the upper airways open during the muscle atonia. The pressure setting is higher than would be needed to keep the upper airways open during non-REM sleep. Although the high pressure during REM sleep helps to reduce the SDB events, the high pressure causes discomfort to the subject. As a result of the discomfort, the subject is aroused. These arousals may lead to sleep fragmentation and a shortage of the amount of REM-sleep. The shortage of REM-sleep may cause day-time sleepiness, physical health issues, and mental health issues.

It is an objective of the invention to determine the effect of the SDB or the SDB therapy has on REM sleep to be able to provide improved SDB therapy.

According to a first aspect, there is provided a computer program product, comprising instructions which, when executed by a processing system, cause the processing system to carry out a method for determining a sleep characteristic of a subject during a sleep session. The method comprises receiving sleep stage information representative of sleep stages of the subject during the sleep session; and receiving respiratory event information representative of respiratory events of the subject during the sleep session. The method comprises determining a first ratio between a REM value and a NREM value. The REM value and the NREM value are based on the sleep stage information and the respiratory event information. The REM value is representative of a number of respiratory events during the REM time per unit of time. The REM time is representative of a time the subject is asleep in a Rapid Eye Movement sleep stage during the sleep session. The NREM value is representative of a number of respiratory events during the NREM time per unit of time. The NREM time is representative of a time the subject is asleep in a non-Rapid Eye Movement sleep stage during the sleep session. The method comprises determining whether the first ratio exceeds a first threshold. The method comprises determining a REM occurrence value representative of an amount of REM time during the sleep session. The method comprises determining whether the REM occurrence value exceeds a second threshold.

The first ratio indicates the ratio between the number of respiratory events during REM sleep and the number of respiratory events during NREM sleep per unit of time. In case the first ratio exceeds the first threshold, i.e., when the number of respiratory events during REM sleep is substantially higher than the number of respiratory events during NREM sleep per unit of time, the subject has a REM-dominant SDB. In another step, it is determined whether the subject has sufficient REM sleep or is REM-deprived. By determining the REM occurrence value, the amount of REM sleep is determined. In case the REM occurrence exceeds the second threshold, i.e., the amount of REM sleep is more than a minimum healthy amount of REM sleep, the subject is not REM-deprived. In that case, the SDB therapy can be adjusted to focus on the reduction of the SDB events during REM sleep. However, in case the REM occurrence does not exceed the second threshold, i.e., the amount of REM sleep is less than a minimum healthy amount of REM sleep, the subject is REM-deprived. In that case, the SDB therapy can be adjusted to balance between the reduction of the SDB events during REM sleep and increasing the amount of REM sleep. For example, the SDB therapy may allow for more SDB events to occur if this leads to more comfort for the subject. The increase in comfort helps to improve the amount of REM sleep. The first ratio and the REM occurrence value are sleep characteristics that provide information about the subject. By making use of these sleep characteristics, improved SDB therapy is obtained.

The sleep stage information is, for example, generated based on neurological signals of the subject. For example, the neurological signals are obtained via polysomnography (PSG), via electroencephalography (EEG), via electrooculography (EOG), via electromyography (EMG) or any combination of these. A sensor adapted to generate a sensor signal based on the neurological signals is, for example, mounted on a wearable device for the head or face, such as a headband. The sleep stage information is based on the neurological signals via use of an automated sleep stage classifier or via manual annotation.

In addition or alternatively to using neurological signals, the sleep stage information is, for example, generated based on a surrogate measure of sleep. For example, the surrogate measure of sleep is based on changes in autonomic nervous system activity of the subject. A change in the autonomic nervous system activity is, for example, detected based on cardiac signals obtained with an appropriate sensor such as a reflective photoplethysmography (PPG) sensor, a transmissive PPG sensor or a remote PPG sensor, a ballistocardiographic sensor, or a seismocardiographic sensor. The reflective PPG sensor is, for example, arranged on the wrist or the face of the subject. The transmissive PPG sensor is, for example, arranged on the finger of the subject. The remote PPG sensor comprises, for example, an infrared camera. The ballistocardiographic sensor comprises, for example, an accelerometer or gyroscope attached to the body of the subject, or for example a pressure sensor mounted in the mattress or bed of the subject. The seismocardiographic sensor comprises, for example, an accelerometer mounted on the chest of the subject. The signals obtained by one or more of these sensors are, for example, used as input to a machine learning model trained to infer sleep stages. The input is, for example, based on manually crafted features correlating with sleep stages, such as a feature describing heart rate variability, or a feature of a time series describing heart rate progression during sleep (e.g. instantaneous heart rate). The input is, for example, input as raw data to the machine learning model.

In addition or alternatively to determining the sleep stage information as mentioned above, the sleep stage information is, for example, based on respiratory activity. Respiratory activity of the subject is indicative of changes in autonomic nervous system activity associated with different sleep stages. Respiratory activity is, for example, measured with a sensor adapted to measure airflow or adapted to measure chest movements. For example, a sensor adapted to measure airflow comprises an oral cannula, a nasal cannula and/or a thermistor. For example, a sensor adapted to measure chest movements comprises a respiratory inductance plethysmography belt to be worn around the thorax of the abdomen. For example, the sensor adapted to measure respiratory activity comprises a pressure sensor mounted on the bed or mattress. For example, the sensor adapted to measure respiratory activity comprises a Doppler radar positioned near the subject. For example, the sensor for measuring respiratory activity comprises an accelerometer or a gyroscope mounted on the thorax, the abdomen and/or sternum of the subject.

The respiratory event information is, for example, information associated with sleep disordered breathing (SDB) and/or OSA. For example, the respiratory example is based on a combination of airflow, respiratory effort and oxygen saturation. For example, the airflow is detected using an airflow sensor or a pressure sensor. For example, the respiratory effort is detected using an accelerometer arranged at the chest or abdomen of the subject. For example, the oxygen saturation is detected using a PPG sensor. For example, the respiratory event information is based on surrogate measurements, such as based on cardiac changes and/or respiratory changes. For example, the sensor that detects cardiac signals to detect changes in the autonomic nervous system activity is used to generate at least part of the respiratory event information. For example, the sensor that detects respiratory activity is used to generate at least part of the respiratory event information.

For example, the respiratory event information comprises information about respiratory events while the subject is undergoing SDB therapy. Such respiratory events may be referred to as "residual" respiratory events. These respiratory events occur despite the SDB therapy being delivered. The residual respiratory events are, for example, a result of an improperly titrated therapy, or a worsening of the SDB condition. In the case of PAP therapy, residual respiratory events are representative of insufficient PAP therapy pressure to keep the airway open during the entire sleep session. In another example, the respiratory event information comprises information about respiratory events while the subject is not undergoing any SDB therapy.

The number of respiratory events comprise, for example, the number of times the subject stops breathing during a certain period while sleeping. The stopping of breathing is an apnea event. The number of respiratory events comprise, for example, the number of times the subject has a reduced breathing during a certain period while sleeping. The reduction of breathing is a hypopnea event. The certain period is, for example, at least 5 or at least 10 or at least 15 seconds.

The REM time is representative of the time the subject is asleep during a REM sleep stage. In the following example, the subject has five periods of REM sleep during the sleep session. The subject has NREM sleep stages in between the five periods of REM sleep. The REM time is the total time of the five periods of REM sleep.

The NREM time is representative of the time the subject is asleep during a NREM sleep stage. In the following example, the subject has six periods of NREM sleep during the sleep session. The subject has REM sleep stages in between the six periods of NREM sleep. The NREM time is the total time of the six periods of NREM sleep.

The non-REM sleep stage comprises, for example, a single sleep stage representative of a non-REM sleep stage. In another example, the non-REM sleep stage comprises at least one light sleep stage, such as N1 and N2. For example, the non-REM sleep stage comprises at least one deep sleep stage, such as N3.

The REM value is representative of the number of respiratory events during the REM time per unit of time. For example, the unit of time is hours or minutes. This way, the REM value represents a number of respiratory events per hour or per minute of REM time. Similarly, the NREM value is representative of the number of respiratory events during the NREM time per unit of time. This way, the NREM value represents a number of respiratory events per hour or per minute of NREM time.

The REM occurrence value is representative of the amount of REM time during the sleep session. For example, the amount of REM time is an absolute time, such as a number of minutes or a number of hours. In another example, the amount of REM time is relative to an amount of NREM time, such as expressed as a percentage of NREM time. In another example, the amount of REM time is relative to an amount of recording time, such as expressed as a percentage of recording time. For example, the subject is awake during part of the recording time. The recording time is the time during which at least one of the sleep stage information and the respiratory event information is obtained from the subject. In another example, the amount of REM time is relative to an amount of bedtime, such as expressed as a percentage of bedtime. The bedtime is the time during which the subject is in bed. For example, the subject is awake during part of the bedtime.

For example, the method comprises determining the REM value based on the sleep stage information and the respiratory event information. For example, the method comprises determining a NREM value based on the sleep stage information and the respiratory event information.

In an embodiment, the REM value is representative of an apnea-hypopnea index (AHI) during the REM time. The NREM value is representative of an apnea-hypopnea index (AHI) during the NREM time. The first threshold is representative of the apnea-hypopnea index (AHI) during the REM time being at least two times greater than the apnea-hypopnea index (AHI) during the NREM time.

According to this embodiment, the AHI during the REM time is used to represent the number of respiratory events during the REM. The AHI during the NREM time is used to represent the number of respiratory events during the NREM time. The AHI is a well-known defined metric to represent the number of respiratory events over a period. As a result, REM value and the NREM value provide an accurate representation of the sleep condition of the subject. In case the AHI during REM is at least two times greater than the AHI during NREM, the subject has substantially more respiratory events during REM sleep than during NREM sleep per unit of time. This provides information to help to provide SDB therapy to improve the AHI during REM sleep. In case the AHI during REM is less than two times greater than the AHI during NREM, the subject does not have substantially more respiratory events during REM sleep than during NREM sleep. Based on this information, SDB therapy may be provided to improve the AHI independent of sleep stages. Optionally, the first threshold includes in addition that the AHI during the REM time needs to be at least 5 respiratory events per hour. In case the AHI during the REM time is at least two times greater than the AHI during NREM, and the AHI during REM time is at least 5 respiratory events per hour, the subject has substantially more respiratory events during REM sleep than during NREM sleep. In case either the AHI during the REM time is not at least two times greater than the AHI during the NREM time, or the AHI during the REM time is less than 5 respiratory events per hour, the subject does not have substantially more respiratory events during REM sleep than during NREM sleep.

In an embodiment, the REM occurrence value is representative of a second ratio. The second ratio is between the REM time and a total sleep time of the sleep session.

According to this embodiment, the second ratio is based on the following. The REM time is determined as mentioned above. The periods of REM sleep during the sleep time are added to determine the REM time. Further, the total sleep time is determined. In case the subject slept for a continuous period, the total sleep time is equal to the continuous period. In case the subject woke up during the sleep session, the multiple periods of sleep are added to determine the total sleep time. For example, the total sleep time is determined by subtracting any awake time during the sleep session from the duration of the sleep session. The awake time comprises sleep onset latency (SOL) and wake after sleep onset (WASO).

In an embodiment, the second ratio is representative of the REM time being less than 25%, or less than 20%, or less than 15% of the total sleep time of the sleep session.

According to this embodiment, the REM occurrence exceeds the second threshold in case the REM time is more than 15%, or more than 20%, or more than 25% of the total sleep time. By having this amount of REM time, the subject has sufficient REM sleep to be healthy. In case the REM time is less than 25%, or less than 20%, or less than 15% of the total sleep time of the sleep session, the subject is REM-deprived. In that case, the REM occurrence value does not exceed the second threshold. This way, based on the first ratio exceeding the first threshold, and based on the REM occurrence value not exceeding the second threshold, the SDB therapy can be adjusted to improve REM sleep.

In an embodiment, the REM occurrence value is representative of a third ratio. The third ratio is between the number of respiratory events during the REM time and the number of respiratory events during the NREM time.

According to this embodiment, the REM occurrence value is based the number of respiratory events during REM. In case the first ratio exceeds the first threshold, the REM value is representative of a high number of respiratory events during the REM time. Because the REM value is based on the number of events per unit of time, the REM value may be high for two different scenarios. In the first scenario, the subject has a normal amount of REM time, and has many respiratory events during the REM time. In the second scenario, the subject has a short amount of REM time, and has only a few respiratory events during the short REM time. So by looking at the number of respiratory events during the REM time, a measure for the amount of REM time is determined. The third ratio is also based on the number of respiratory events during the NREM time to provide a reference for the number of respiratory events during the REM time.

In an embodiment, the second threshold is representative of the number of respiratory events during the REM time being 0.5 times the number of respiratory events during the NREM time.

According to this embodiment, the second threshold is set to the number of respiratory events during the REM time being 0.5 times the number of respiratory events during the NREM time. The value of 0.5 is based on that a healthy amount of REM sleep is about 25% of the total sleep time. The subject has a REM-dominant SDB in case the number of respiratory events per unit of time is at least two times greater than the number of respiratory events per unit of time. Multiplying the 25% by two (of the at least two times greater) gives the value of 0.5. So in case the first ratio is exceeded, and the third ratio is less than 0.5, then the amount of REM time is less than the healthy amount of 25% of the total sleep time.

For example, the second threshold is representative of the number of respiratory events during the REM time being 0.4 times the number of respiratory events during the NREM time. The value of 0.4 is based on that a minimum healthy amount of REM sleep is about 20% of the total sleep time. Multiplying the 20% by two (of the at least two times greater) gives the value of 0.4. So in case the first ratio is exceeded, and the third ratio is less than 0.4, then the amount of REM time is less than the healthy amount of 20% of the total sleep time.

In an embodiment, the first ratio is based on a probability difference representative of a difference between a probability of an occurrence of a respiratory event during the REM time and a probability of an occurrence of a respiratory event during the NREM time. The second ratio is based on the probability difference. The first threshold is representative of a lower threshold value of the probability difference. The second threshold is representative of an upper threshold value of the probability difference.

According to this embodiment, in case the first ratio does not exceed the lower threshold value, the subject has the same or about the same probability of having a respiratory event during REM time as during NREM time. So in that case the subject, the subject has non-REM-specific OSA. In case the first ratio exceeds the lower threshold value, the subject has a higher probability of having a respiratory event during REM time than during NREM time. So in that case the subject has REM-OSA or the subject has REM-OSA and REM-deprivation. In case the second ratio exceeds the upper threshold, the subject has a substantially higher probability of having a respiratory event during REM time than during NREM time. In this case, the subject has REM-OSA, but does not have REM-deprivation. In case the second ratio does not exceed the upper threshold, the subject has both REM-OSA and REM-deprivation. The subject with both REM-OSA and REM-deprivation has a higher probability of having a respiratory event during REM time than during NREM time, but this probability is not as high as for REM-OSA. The probability is not as high, because the subject has less respiratory events during the REM time per unit of time, and the REM time is shorter than a healthy amount.

In an embodiment, the probability difference comprises at least one of a risk ratio and an odds ratio. The lower threshold value is 2. The upper threshold value is 3. The lower threshold value of 2 is indicative of that the subject has a substantial higher probability of having a respiratory event during REM time than during NREM time. In case the probability difference exceeds the upper threshold value of 3, there is a clear correlation between REM sleep and the respiratory events. Based on this clear correlation, it may be determined that the subject has REM-OSA. However, a subject having both REM-OSA and REM-deprivation does not have such a clear correlation. As a result, the value of the odds ratio or the risk ratio is between the lower threshold value and the upper threshold value.

In an embodiment, the method comprises classifying the subject as having REM-related obstructive sleep apnea (REM-OSA) based on the first ratio exceeding the first threshold and based on the REM occurrence value exceeding the second threshold; and classifying the subject as having both REM-related obstructive sleep apnea (REM-OSA) and REM-deprivation based on the first ratio exceeding the first threshold and based on the REM occurrence value not exceeding the second threshold.

According to this embodiment, in case it is classified that the first ratio exceeds the first threshold, this means that the subject has substantially more respiratory events per unit of time during the REM sleep than during the NREM sleep. In case the REM occurrence value exceeds the second threshold, it is determined that the subject has a sufficient amount of REM sleep. Based on both the substantially more respiratory events per unit of time during the REM sleep, and based on the sufficient amount of REM sleep, it can be determined that the subject has REM-OSA. For another subject, in case it is classified that the first ratio exceeds the first threshold, this means that the subject has substantially more respiratory events per unit of time during the REM sleep than during the NREM sleep. In case the REM occurrence value does not exceed the second threshold, it is determined that the subject has an insufficient amount of REM sleep. Based on both the substantially more respiratory events per unit of time during the REM sleep, and based on the insufficient amount of REM sleep, it can be determined that the subject has both REM-OSA and REM-deprivation.

In an embodiment, the method comprises classifying the subject as having non-REM-specific OSA based on the first ratio not exceeding the first threshold.

According to this embodiment, the first ratio does not exceed the first threshold in case the first ratio between the REM value and the NREM value is presentative of that the REM value is not substantially greater than the NREM value. The subject has about the same number of respiratory events per unit of time during REM sleep as well as during NREM sleep. Therefore, the subject is classified as having non-REM-specific OSA. Non-REM-specific means in the context of the invention that the OSA is not substantially worsened by REM sleep.

In an embodiment, the method comprises receiving from a sensor system at least one sensor signal representative of the sleep stage information and the respiratory event information.

According to this embodiment, the sensor system comprises, for example, any one of the sensors mentioned above. For example, one sensor generates a sensor signal representative of the sleep stage information, whereas another sensor generates a sensor signal representative of the respiratory event information. For example, a single sensor generates both the sensor signal representative of the sleep stage information and the sensor signal representative of the respiratory event information. For example, multiple sensors generate multiple sensor signals that together generate sleep stage information or the respiratory event information.

In an embodiment, the method comprises providing an output signal representative of an action based on whether the first ratio exceeds the first threshold and based on whether the REM occurrence value exceeds the second threshold.

According to this embodiment, the output signal represents an action. Depending on whether the first ratio exceeds the first threshold and on whether the REM occurrence value exceeds the second threshold, the action is different.

In case the first ratio does not exceed the first threshold, the subject does not have substantially more respiratory events per unit of time during REM sleep than during NREM sleep. The action is, for example, to adjust SDB therapy to minimize the number of respiratory events per unit of time.

In case the first ratio exceeds the first threshold, and the REM occurrence value exceeds the second threshold, the subject has substantially more respiratory events per unit of time during REM sleep than during NREM sleep, thus the subject has REM-dominant OSA. In addition, the subject has sufficient REM sleep as indicated by the REM occurrence value exceeding the second threshold. The action is, for example, to adjust SDB therapy to minimize the number of respiratory events during REM sleep. The action is, for example, to provide more intense SDB therapy during REM sleep than during NREM sleep.

In case the first ratio exceeds the first threshold, and the REM occurrence value does not exceed the second threshold, the subject has substantially more respiratory events per unit of time during REM sleep than during NREM sleep, thus the subject has REM-dominant OSA. As the REM occurrence value does not exceed the second threshold, the subject has insufficient REM sleep. The action is, for example, to improve REM time. For example, the action is to reduce the intensity of the SDB therapy. The reduced intensity improves comfort for the subject, resulting in improved REM time at the expense of more respiratory events. For example, the intensity is set to balance an acceptable amount of REM time and an acceptable number of respiratory events. For example, the action is to use or to suggest using sleep medication such as hypnotics. Sleep medication, and especially hypnotics, are able to alter or improve sleep architecture. This way, sleep medication can be used to improve the amount of REM time.

In an embodiment, the action comprises suggesting adjusting a sleep disordered breathing therapy performed on the subject or start performing another type of sleep disordered breathing therapy on the subject.

For example, the action is to start SDB therapy or the change to a different type of SDB therapy. For example, a type of SDB therapy is PAP therapy or positional sleep therapy or mandibular advancement therapy, or surgical therapy. During PAP therapy, a pressure applied to the airway of the subject prevents or reduces collapse of the airway. During positional sleep therapy, feedback is provided to the subject in case the subject is in a supine position. The feedback prompts the subject to change to a different position than the supine position. As the supine position worsens the SDB for some subjects, changing to a different position helps to reduce the SDB. During mandibular advancement therapy, a device is placed to pull the jaw of the subject outward. This tightens the soft tissue and muscles of the upper airway to prevent or reduce obstruction of the airway during sleep. During surgical therapy, for example, soft tissue in the mouth or throat is removed, such as with UPPP surgery.

For example, the action is to adjust the SDB therapy. For example, PAP therapy is adjusted by reducing the pressure of a pressurized airflow to the subject. By reducing the pressure, the comfort for the subject is improved, resulting in an increase in REM time. For example, PAP therapy is adjusted by increasing the pressure of the pressurized airflow to the subject during REM sleep. By increasing the pressure, the number of respiratory events during REM sleep is reduced.

For example, positional therapy is adjusted by limiting the feedback to the subject when the subject is in the supine position. For example, the feedback is limited by delaying the feedback for several minutes. During this time without feedback, the subject is able to have undisturbed REM sleep. For example, the feedback is only provided in case an SDB event is detected. This allows the subject to have undisturbed REM sleep in case there is no SDB event, while sleeping supine. For example, mandibular advancement is adjusted by reducing the extension of the jaw by the mandibular advancement device. By reducing the extension, the comfort of the subject is improved. The improved comfort improves REM time at the expense of an increase of respiratory events. The extension of the jaw is, for example, set to obtain both an acceptable REM time as well as an acceptable number of respiratory events.

In an embodiment, the method comprises providing the output signal for use in a respiratory support device for providing pressurized air to the subject. The action comprises adjusting a setting of the respiratory support device.

According to this embodiment, the setting of the respiratory device is adjusted to improve the SDB therapy to the subject. For example, the setting relates to a pressure of the pressurized air to the subject, a humidity of the pressurized air to the subject, or a pressure profile of the pressurized air to the subject. For example, the pressure profile has a high pressure during a first part of the sleep session, and a low pressure during a second part of the sleep session. The high pressure is higher than the low pressure. The first part is earlier than the second part. REM sleep occurs more later in the sleep session, so by having the high pressure during the first part of the sleep session, the number of respirator events is reduced, while the low pressure during the second part of the sleep session improves the amount of REM time.

According to a second aspect of the invention, there is provided a respiratory support device for providing pressurized air to a subject, the respiratory support device comprising: a pressure source adapted to generate the pressurized air; a processing system configured to execute the computer program product of the first aspect of the invention; and a sensor input adapted to receive from a sensor system at least one sensor signal representative of the sleep stage information and the respiratory event information. The processing system is configured to adjust a setting of the respiratory support device based on whether the first ratio exceeds the first threshold and based on whether the REM occurrence value exceeds the second threshold.

According to the second aspect of the invention, the respiratory support device is able to adjust the setting to reduce the number of respiratory events while allowing the subject to have sufficient REM sleep.

In an embodiment, the processor system is configured to adjust the setting by reducing a pressure of the pressurized air.

By reducing the pressure of the pressurized air, the comfort for the subject is increased. The increase in comfort improves the amount of REM time. Even though the reduced pressure may result in an increase of respiratory events, this increase of respiratory events may be acceptable, depending on the condition of the subject. A subject may benefit more from sufficient REM sleep and some respiratory events than from insufficient REM sleep with no respiratory events.

In an embodiment, the processing system is configured to adjust the setting during the sleep session based on at least one of a REM time already spent during the sleep session and an expected REM time for a remainder of the sleep session.

According to this embodiment, the REM time already spent and the expected REM time for the remainer of the sleep session determine the adjustment of the setting. For example, the total sleep time is known or estimated for the subject, such as based on historical sleep data. About 20-25% of the total sleep time should be REM time to have sufficient REM sleep. While the sleep session progresses, the processing system monitors the amount of REM time already spent. In case the REM time already spent is more than expected based on the total sleep time, the processing system adjusts the setting to intensify the SDB therapy to decrease the number of respiratory events. This adjustment may, however, reduce the amount of REM sleep following the adjustment. In case the REM time already spent is less than expected based on the total sleep time, the processing system adjusts the setting to reduce the intensity of the SDB therapy to increase the REM time. This adjustment may, however, increase the number of respiratory events following the adjustment.

According to a third aspect of the invention, there is provided a therapy device for providing sleep positional therapy. The therapy device comprises a position sensor, a sensory stimulator, and a processing system. The position sensor is adapted to generate a position signal representative of whether the subject is in a target position. The sensory stimulator is adapted to provide sensory stimulation. The processing system is configured to execute the computer program product of any one of the preceding claims. The processing system is configured to control the sensory stimulator to provide stimulation based on: the position signal, whether the first ratio exceeds the first threshold, and whether the REM occurrence value exceeds the second threshold.

For some subjects, the SDB events become worse when the subject is in a target position, such as in a supine position. The therapy device provides sleep positional therapy by providing sensory stimulation in case the subject is in the target position. The sensory stimulation prompts the subject to change position. The sensor stimulation stops when the subject is no longer in the target position. By controlling the sensory stimulator based on the position signal, whether the first ratio exceeds the first threshold, and whether the REM occurrence value exceeds the second threshold, the therapy device is able to reduce respiratory events while allowing the subject to have improved REM sleep. For example, the sensory stimulation is not provided during REM sleep, or is provided during REM sleep only after a waiting period to allow the subject to have an increased REM time.

The sensory stimulator is adapted to provide sensory stimulation, such as vibrations, or light, or sound. For example, the sensor stimulation comprises tactile feedback.

In an embodiment, the processing system is configured to: control the sensory stimulator to provide stimulation in case the subject is in the target position and has a respiratory event, and control the sensory stimulator not to provide stimulation in case the subject is in the target position and does not have a respiratory event.

In a fourth aspect of the invention, there is provided a method for determining a sleep characteristic of a subject during a sleep session, the method comprising receiving sleep stage information representative of sleep stages of the subject during the sleep session, and receiving respiratory event information representative of respiratory events of the subject during the sleep session. The method comprises determining a first ratio between a REM value and a NREM value. The REM value and the NREM value are based on the sleep stage information and the respiratory event information. The REM value is representative of a number of respiratory events during a REM time per unit of time. The REM time is representative of a time the subject is asleep in a Rapid Eye Movement sleep stage during the sleep session. The NREM value is representative of a number of respiratory events during a NREM time per unit of time. The NREM time is representative of a time the subject is asleep in a non-Rapid Eye Movement sleep stage during the sleep session. The method comprises determining whether the first ratio exceeds a first threshold. The method comprises determining a REM occurrence value representative of an amount of REM sleep during the sleep session, and determining whether the REM occurrence value exceeds a second threshold.

In an embodiment, the method comprises determining the REM value based on the sleep stage information and the respiratory event information, and determining the NREM value based on the sleep stage information and the respiratory event information.

In an embodiment, the REM value is representative of an apnea-hypopnea index (AHI) during the REM time. The NREM value is representative of an apnea-hypopnea index (AHI) during the NREM time. The first threshold is representative of the apnea-hypopnea index (AHI) during the REM time being at least two times greater than the apnea-hypopnea index (AHI) during the NREM time.

In an embodiment, the REM occurrence value is representative of a second ratio. The second ratio is between the REM time and a total sleep time of the sleep session. Optionally, the second ratio is representative of the REM time being less than 20%, or less than 15% of the total sleep time of the sleep session.

In an embodiment, the REM occurrence value is representative of a third ratio. The third ratio is between the number of respiratory events during the REM time and the number of respiratory events during the NREM time. Optionally, the second threshold is representative of the number of respiratory events during the REM time being 0.5 times the number of respiratory events during the NREM time.

In an embodiment, the first ratio is based on a probability difference representative of a difference between a probability of an occurrence of a respiratory event during the REM time and a probability of an occurrence of a respiratory event during the NREM time. The second ratio is based on the probability difference. The first threshold is representative of a lower threshold value of the probability difference. The second threshold is representative of an upper threshold value of the probability difference. Optionally, the probability difference comprises at least one of a risk ratio and an odds ratio. The lower threshold value is 2. The upper threshold value is 3.

In an embodiment, the method comprises: classifying the subject as having REM-related obstructive sleep apnea (REM-OSA) based on the first ratio exceeding the first threshold and based on the REM occurrence value exceeding the second threshold; classifying the subject as having both REM-related obstructive sleep apnea (REM-OSA) and REM-deprivation based on the first ratio exceeding the first threshold and based on the REM occurrence value not exceeding the second threshold.

In an embodiment, the method comprises: classifying the subject as having non-REM-specific OSA based on the first ratio not exceeding the first threshold.

In an embodiment, the method comprises receiving from a sensor system at least one sensor signal representative of the sleep stage information and the respiratory event information.

In an embodiment, the method comprises providing an output signal representative of an action based on whether the first ratio exceeds the first threshold and based on whether the REM occurrence value exceeds the second threshold.

In an embodiment, the action comprises suggesting adjusting a sleep disordered breathing therapy performed on the subject or start performing another type of sleep disordered breathing therapy on the subject.

In an embodiment, the method comprises providing the output signal for use in a respiratory support device, wherein the action comprises adjusting a setting of the respiratory support device.

BRIEF DESCRIPTION OF THE DRAWINGS

Exemplary embodiments will now be described, by way of example only, with reference to the following Figures, in which:

FIG. 1 depicts a first embodiment according to the invention;
FIG. 2 depicts a detail of the first embodiment;
FIG. 3 depicts an example of a part of a sleep session;
FIG. 4 depicts a graph representing respiratory events in NREM and REM for a group of subjects;
FIG. 5 depicts a method according to the first embodiment of the invention;
FIG. 6 depicts the use of the risk ratio according to a second embodiment;
FIG. 7 depicts the use of the odds ratio according to a third embodiment;
FIG. 8 depicts a second embodiment according to the invention;

DETAILED DESCRIPTION OF EMBODIMENTS

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

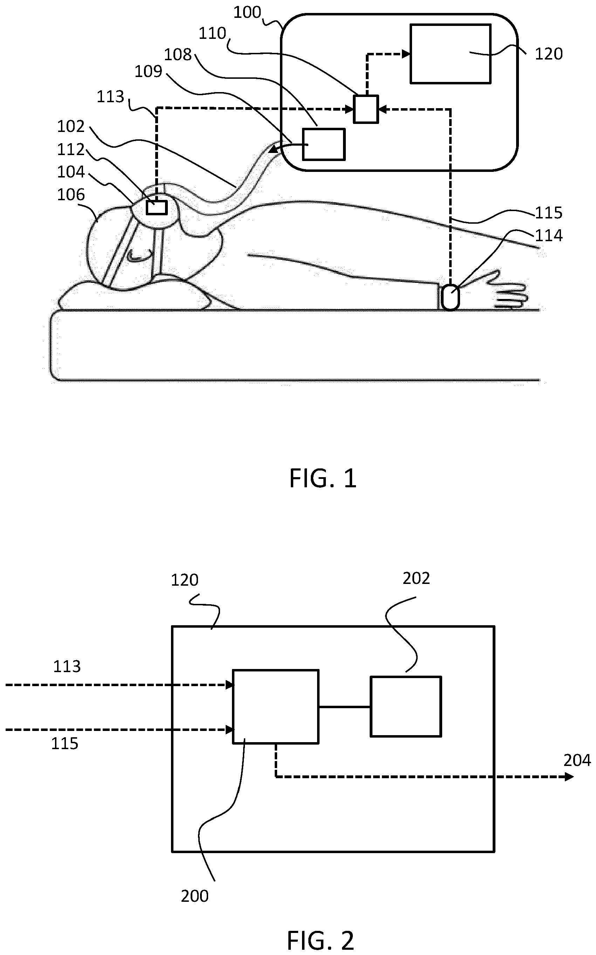

FIG. 1 depicts a first embodiment according to the invention. There is provided a respiratory support device 100 for providing pressurized air to a subject 106. The respiratory support device 100 comprises a pressure source 108, a sensor input 110, and a processing system 120. The pressure source 108 is adapted to generate the pressurized air 109. The pressurized air 109 is delivered to the subject 106 via an air delivery tube 102 connected to a patient interface 104. A respiratory sensor 112 is located in the patient interface 104. Alternatively, the respiratory sensor 112 is located in the air delivery tube 102 or in the respiratory support device 100. The respiratory sensor 112 generates a sensor signal 113 representative of the respiratory event information. A cardiac sensor 114 is located at the wrist of the subject 106. The cardiac sensor 114 generates a sensor signal 115 representative of the sleep stage information. The respiratory sensor 112 and the cardiac sensor 114 form a sensor system. The sensor input 110 is adapted to receive from the sensor system the sensor signal 115 representative of the sleep stage information and the sensor signal 113 representative of the respiratory event information.

The processing system 120 is coupled to the sensor input 110 to receive the sleep stage information and the respiratory event information. The processing system 120 is configured to execute a computer program to carry out a method for determining a sleep characteristic of the subject 106 during a sleep session.

FIG. 2 depicts a detail of the first embodiment. The processing system 120 comprises a processing unit 200, and a memory 202. The processing unit 200 is configured to receive the sleep stage information and the respiratory event information via sensor signals 113 and 115. The memory 202 is connected to the processing unit 200. The memory 202 stores the computer program product. Optionally, the processing unit 200 provides an output signal 204.

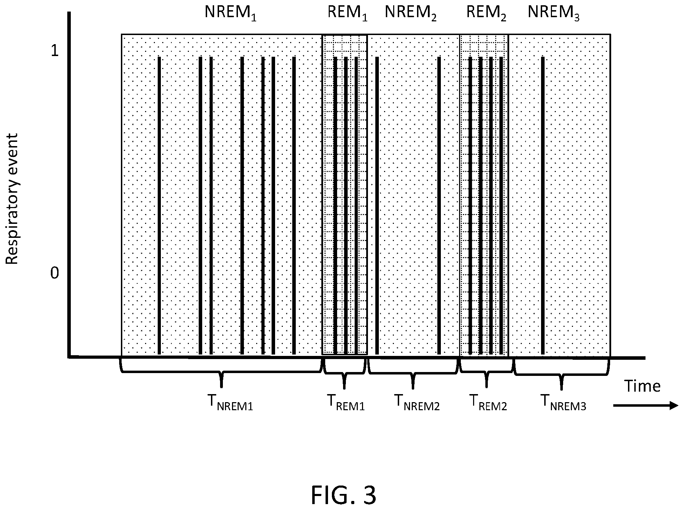

FIG. 3 depicts an example of a part of a sleep session. The x-axis indicates time in the sleep session. The y-axis indicates the presence of respiratory events. In the graph, it is depicted in which sleep stage the respiratory events occur.

During the sleep session, while the subject 106 is sleeping, the subject 106 is in either a REM sleep stage or a NREM sleep stage. FIG. 3 depicts a part of the sleep session, in which the subject 106 is three times in a NREM sleep stage ($NREM_{1-3}$) and two times in a REM sleep stage ($REM_{1-2}$). This part of the sleep session extends, for example, for several minutes, whereas the complete sleep session extends, for example, for 6-8 hours.

During the first NREM sleep stage, $NREM_1$, the subject 106 experiences 7 respiratory events as indicated with the vertical lines. During the first REM sleep stage, $REM_1$, the subject 106 experiences 3 respiratory events as indicated with the vertical lines. During the second NREM sleep stage, $NREM_2$, the subject 106 experiences 2 respiratory events as indicated with the vertical lines. During the second REM sleep stage, $REM_2$, the subject 106 experiences 4 respiratory events as indicated with the vertical lines. During the third NREM sleep stage, $NREM_3$, the subject 106 experiences 1 respiratory event as indicated with the vertical line.

The duration of $NREM_1$ is $T_{NREM1}$. The duration of $NREM_2$ is $T_{NREM2}$. The duration of $NREM_3$ is $T_{NREM3}$. The duration of $REM_1$ is $T_{REM1}$. The duration of $REM_2$ is $T_{REM2}$. The duration of each $T_{NREM1-3}$ and $T_{REM1-2}$ is, for example, expressed in seconds or minutes.

REM time is representative of the time the subject 106 is asleep in a REM sleep stage during the sleep session. So REM time is the sum of $T_{REM1}$ and $T_{REM2}$ and any other time the subject 106 is in the REM sleep stage during the sleep session. NREM time is representative of the time the subject 106 is asleep in a NREM sleep stage during the sleep session. So NREM time is the sum of $T_{NREM1}$-$T_{NREM3}$ and any other time the subject 106 is in the NREM sleep stage during the sleep session.

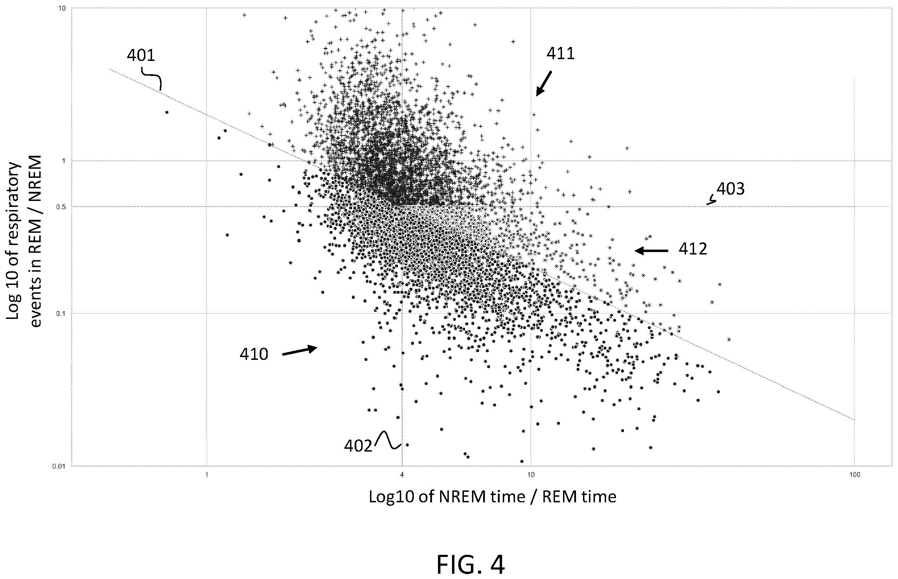

FIG. 4 depicts a graph representing the number of respiratory events in NREM and REM for a group of 7132 subjects. For each of the subjects, the number of respiratory events during NREM, the number of respiratory events during REM, the REM time, and the NREM time are determined. The REM time, and the NREM time are determined based on sleep stage information. The number of respiratory events during the NREM time, and the number of respiratory events during the REM time are based on respiratory event information.

The x-axis represents the ratio between the NREM time and the REM time in a log10 scale. The y-axis represents the ratio between the number of respiratory events during the REM time and the number of respiratory events during the NREM time. Each subject is represented in the graph as either a dot ".", a cross "+" or an 'x'. The difference between the dot, the cross and 'x' is explained further.

Based on the sleep stage information representative of sleep stages of the subjects, and based on the respiratory event information of the subjects, a REM value and a NREM value are determined. The REM value is representative of a number of respiratory events during a REM time per unit of time. The REM value is based on the number of respiratory events during REM, and based on the REM time. The REM time is representative of a time the subject 106 is asleep in a Rapid Eye Movement sleep stage during the sleep session. The NREM value is representative of a number of respiratory events during a NREM time per unit of time. The NREM value is based on the number of respiratory events during NREM, and based on the NREM time The NREM time is representative of a time the subject 106 is asleep in a non-Rapid Eye Movement sleep stage during the sleep session. Then a first ratio between the REM value and the NREM value is determined. The first ratio is determined based on the information from FIG. 4, i.e., the ratio between the number of respiratory events in REM and NREM, and the ratio between the NREM time and the REM time. Then it is determined whether the first ratio exceeds a first threshold 401. For example, the first threshold 401 is calculated by:

$$\frac{NREM\ time}{REM\ time} * \frac{respiratory\ events\ in\ REM}{respiratory\ events\ in\ NREM} = 2 \tag{1}$$

The first threshold 401 separates the subjects that have substantially more respiratory events during REM than during NREM from the subjects that do not have substantially more respiratory events during REM than during NREM. The subjects that do not have substantial more respiratory events during REM than during NREM are indicated with the dots below the first threshold 401. These subjects have non-REM-specific OSA as indicated with area 410.

The subjects that have substantially more respiratory events during REM than during NREM are indicated with the crosses and 'x' above the first threshold 401. For these subjects, the REM occurrence value is determined. The REM occurrence value is representative of an amount of REM time during the sleep session. It is determined whether the REM occurrence value exceeds a second threshold 402.

The second threshold 402 separates the subjects that have sufficient REM time from subjects that have insufficient REM time. The subjects that have sufficient REM time are located on the left side of the second threshold 402. For these subjects, the REM time is sufficiently large compared to the NREM time. The subjects that have insufficient REM time are located on the right side of the second threshold 402. For these subjects, the REM time is small compared to the NREM time.

In an alternative embodiment, the REM occurrence value is representative of a second ratio. The second ratio is between the REM time and a total sleep time of the sleep session. For example, the second threshold 402 is representative of the REM time being les than 25%, or less than 20%, or less than 15% of the total sleep time of the sleep session.

Alternatively or in addition, the REM occurrence value is representative of a third ratio. The third ratio is between the number of respiratory events during the REM time and the number of respiratory events during the NREM time. Note that this is not the number of respiratory events per unit of time, but the absolute count of the number of respiratory events during the REM time and the absolute count of the number of respiratory events during the NREM time. Line 403 depicts a third threshold for the third ratio, which separates the subjects that have substantially more respiratory events during REM than during NREM from the subjects that do not have substantially more respiratory events during REM than during NREM. Not having substantially more respiratory events during REM than during NREM, while these subjects have a high number of respiratory events during REM per unit of time is a measure of an insufficient REM time. The subjects that have sufficient REM sleep are located above the third threshold 403. The subjects represented above the third threshold 403 have such a large number of respiratory events during REM time, that these subjects benefit the most from reducing the respiratory events during REM time. Therefore, these subjects benefit from SDB therapy aimed at reducing the number of respiratory events during REM sleep. The subjects that have insufficient REM sleep are located below the third threshold 403. These subjects have a high number of respiratory events during REM time per unit of time, because the REM time is very short. These subjects, however, do not have many respiratory events during REM time in total. Therefore, these subjects benefit from SDB therapy aimed at balancing a reduction of the number of respiratory events during REM sleep and improving REM time.

In this embodiment, the third threshold 403 is representative of the number of respiratory events during the REM time being 0.5 times the number of respiratory events during the NREM time. For example, the third threshold 403 is representative of the number of respiratory events during the REM time being less than 0.5 times the number of respiratory events during the NREM time.

In an embodiment, it is determined that a subject has both REM-OSA and REM-deprivation is based on using the first threshold 401 and the second threshold 402 without using the third threshold 403. In another embodiment it is determined that a subject has both REM-OSA and REM-deprivation is based on using the first threshold 401 and the third threshold 403, without using the second threshold 402.

Based on the first threshold 401 and the third threshold 403, it can be determined which subjects have non-REM-specific OSA, which subjects have REM-OSA, and which subjects have REM-OSA and REM-deprivation. The subjects with non-REM-specific OSA are located below the first threshold 401 in area 410. The subjects with REM-OSA are located above the first threshold 401 and above the third threshold 403 in area 411. The subjects with REM-OSA and REM-deprivation are located above the first threshold 401, and below the third threshold 403 in area 412.

In FIG. 4, there are 7132 subjects represented in total. There are 3458 subjects that have non-REM-specific OSA and are shown in area 410. There are 3010 subjects that have REM-OSA and are shown in area 411. There are 664 subjects that have both REM-OSA and REM-deprivation and are shown in area 412.

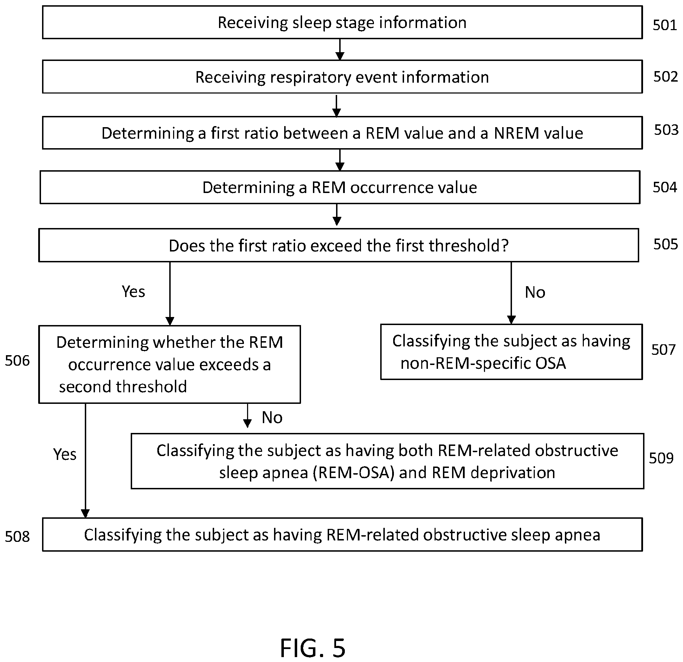

The processing system 120 of the first embodiment is configured to execute a computer program product, comprising instructions which, when executed by the processing system 120, cause the processing system 120 to carry out a method for determining a sleep characteristic of a subject 106 during a sleep session. The method is depicted in FIG. 5. The method comprises in step 501, receiving sleep stage information representative of sleep stages of the subject 106 during the sleep session. The method comprises, in step 502, receiving respiratory event information representative of respiratory events of the subject 106 during the sleep session. The method comprises, in step 503, determining a first ratio between a REM value and a NREM value. The REM value is representative of a number of respiratory events during a REM time per unit of time. The REM time is representative of a time the subject 106 is asleep in a Rapid Eye Movement sleep stage during the sleep session. The NREM value is representative of a number of respiratory events during a NREM time per unit of time. The NREM time is representative of a time the subject 106 is asleep in a non-Rapid Eye Movement sleep stage during the sleep session. Optionally, the method comprises determining the REM value based on the sleep stage information and the respiratory event information. Optionally, the method comprises determining the NREM value based on the sleep stage information and the respiratory event information. The method comprises, in step 504, determining a REM occurrence value representative of an amount of REM sleep during the sleep session. The method comprises, in step 505, determining whether the first ratio exceeds the first threshold 401. The method comprises, in step 506, determining whether the REM occurrence value exceeds the second threshold 402 in case the first ratio exceeds the first threshold.

The method makes use of the information as depicted in FIG. 4. In case the method determines that the first ratio does not exceed the first threshold 401, the subject 106 has non-REM-specific OSA as indicated with area 410. In this case, the method classifies, in step 507, the subject 106 as having non-REM-specific OSA. In case the method determines that the first ratio exceeds the first threshold 401, and that the REM occurrence value exceeds the second threshold 402, the subject 106 has REM-OSA, as indicated with area 411. In this case, the method classifies, in step 508, the subject 106 as having REM-OSA. In case the method determines that the first ratio exceeds the first threshold 401, and that the REM occurrence value does not exceed the second threshold 402, the subject 106 has REM-OSA and REM-deprivation, as indicated with area 412. In this case, the method classifies, in step 509, the subject 106 as having REM-OSA and having REM-deprivation.

In an example, the REM value is representative of an apnea-hypopnea index (AHI) during the REM time. The NREM value is representative of an apnea-hypopnea index (AHI) during the NREM time. The first threshold 401 is representative of the apnea-hypopnea index (AHI) during the REM time being at least two times greater than the apnea-hypopnea index (AHI) during the NREM time. The slope of the first threshold 401 in FIG. 4 is representative of the apnea-hypopnea index (AHI) during the REM time being two times greater than the apnea-hypopnea index (AHI) during the NREM time.

In an example, the REM occurrence value is representative of the second ratio. The second ratio is between the REM time and a total sleep time of the sleep session. Optionally, the second ratio is representative of the REM time being less than 20%, or less than 15% of the total sleep time of the sleep session.

In an example, the REM occurrence value is representative of the third ratio. The third ratio is between the number of respiratory events during the REM time and the number of respiratory events during the NREM time. In case the method determines that the first ratio exceeds the first threshold 401 and that the third ratio exceeds the third threshold 403, the subject 106 has REM-OSA, as indicated with area 411. In case the method determines that the first ratio exceeds the first threshold 401 and that the third ratio does not exceed the third threshold 403, the subject 106 has REM-OSA and REM-deprivation, as indicated with area 412. For example, the third threshold 403 is representative of the number of respiratory events during the REM time being less than 0.5 than the number of respiratory events during the NREM time.

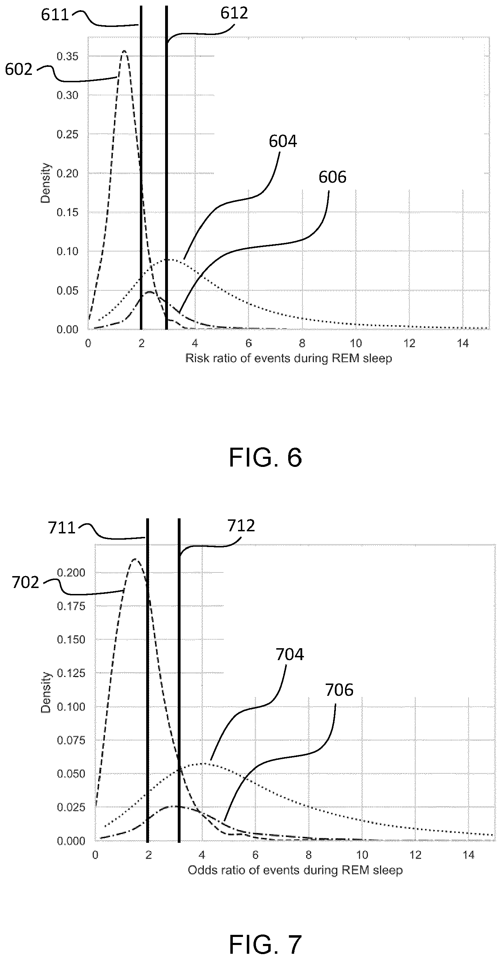

In a second embodiment, the first ratio is based on at least one of a risk ratio and an odds ratio. FIG. 6 depicts the use of the risk ratio according to the second embodiment. FIG. 7 depicts the use of the odds ratio according to the second embodiment. The second embodiment has, for example, the same elements as the first embodiment, except for the following.

The REM occurrence value is based on the least one of a risk ratio and an odds ratio. The at least one of a risk ratio and an odds ratio is representative of a difference between a likelihood of occurrences of respiratory events during the REM time and a likelihood of occurrences of respiratory events during the NREM time. The first threshold 401 is representative of a lower threshold value of the least one of a risk ratio and an odds ratio. The second threshold 402 is representative of an upper threshold value of the least one of a risk ratio and an odds ratio.

The risk ratio, as such, is a well-known statistical parameter to represent the ratio of a probability of an outcome in an exposed group to the probability of an outcome in an unexposed group.

The odds ratio, as such, is a well-known statistical parameter to represent how strongly an event is associated with exposure.

In FIG. 6, the first ratio and the REM occurrence value are based on the risk ratio. The risk ratio indicates the probability of a subject having a respiratory event during REM sleep.

Line 602 is representative of a subject having non-REM-specific OSA. The peak of line 602 indicates the value for the risk ratio of this subject. For this subject, the risk ratio is less than the lower threshold value 611, because this subject has about the same probability or only a slightly higher probability of having a respiratory event during REM sleep than during NREM sleep. So the first ratio does not exceed the first threshold 401, because the risk ratio does not exceed the lower threshold value 611.

Line 604 is representative of a subject having REM-OSA. The peak of line 604 indicates the value for the risk ratio of this subject. For this subject, the risk ratio is higher than the upper threshold value 612, because this subject has a substantially higher probability of having a respiratory event during REM sleep than during NREM sleep. So the first ratio exceeds the first threshold 401, because the risk ratio exceeds the lower threshold value 611. Further, the REM occurrence value exceeds the second threshold 402, because the risk ratio exceeds the upper threshold value 612.

Line 606 is representative of a subject having both REM-OSA and REM-deprivation. The peak of line 606 indicates the value for the risk ratio of this subject. For this subject, the risk ratio is higher than the lower threshold value 611, because this subject has a substantially higher probability of having a respiratory event during REM sleep than during NREM sleep. However, the probability is not as high as represented with line 604, because the subject with both REM-OSA and REM-deprivation has a shortage of REM sleep. So, the REM occurrence value does not exceed the second threshold 402, because the risk ratio does not exceed the upper threshold value 612.

In this embodiment, the lower threshold value is 2, and the upper threshold value is 3.

In FIG. 7, the first ratio and the REM occurrence value are based on the odds ratio. The odds ratio indicates the probability of a subject having a respiratory event during REM sleep.

Line 702 is representative of a subject having non-REM-specific OSA. The peak of line 702 indicates the value for the odds ratio of this subject. For this subject, the odds ratio is less than the lower threshold value 711, because this subject has about the same probability or only a slightly higher probability of having a respiratory event during REM sleep than during NREM sleep. So the first ratio does not exceed the first threshold 401, because the odds ratio does not exceed the lower threshold value 711.

Line 704 is representative of a subject having REM-OSA. The peak of line 704 indicates the value for the odds ratio of this subject. For this subject, the odds ratio is higher than the upper threshold value 712, because this subject has a substantially higher probability of having a respiratory event during REM sleep than during NREM sleep. So the first ratio exceeds the first threshold 401, because the odds ratio exceeds the lower threshold value 711. Further, the REM occurrence value exceeds the second threshold 402, because the odds ratio exceeds the upper threshold value 712.

Line 706 is representative of a subject having both REM-OSA and REM-deprivation. The peak of line 706 indicates the value for the odds ratio of this subject. For this subject, the odds ratio is higher than the lower threshold value 711, because this subject has a substantially higher probability of having a respiratory event during REM sleep than during NREM sleep. However, the probability is not as high as represented with line 704, because the subject with both REM-OSA and REM-deprivation has a shortage of REM sleep. So, the REM occurrence value does not exceed the second threshold 402, because the odds ratio does not exceed the upper threshold value 712.

In this embodiment, the lower threshold value is 2, and the upper threshold value is 3.

The risk ratio and the odds ratio are, for example, used both to improve the accuracy with which it is classified whether the subject has REM-OSA, non-REM-specific OSA or both REM-OSA and REM-deprivation.

In any of the embodiments, the method comprises, for example, receiving from a sensor system at least one sensor signal representative of the sleep stage information and the respiratory event information. The sensor system has, for example, any one or more of the sensors mentioned above.

In any of the embodiments, the method comprises, for example, providing the output signal 204 representative of an action based on whether the first ratio exceeds the first threshold 401 and based on whether the REM occurrence value exceeds the second threshold 402. For example, the action comprises suggesting adjusting a sleep disordered breathing therapy performed on the subject 106 or start performing another type of sleep disordered breathing therapy on the subject 106. For example, the method comprises providing the output signal 204 for use in the respiratory support device 100. The action comprises adjusting a setting of the respiratory support device 100. For example, the processor system is configured to adjust the setting by reducing a pressure of the pressurized air. For example, the processing system 120 is configured to adjust the setting during the sleep session based on at least one of a REM time already spent during the sleep session and an expected REM time for a remainder of the sleep session.

For example, the processing system 120 is configured to adjust a setting of the respiratory support device 100 based on whether the first ratio exceeds the first threshold 401 and based on whether the REM occurrence value exceeds the second threshold 402.

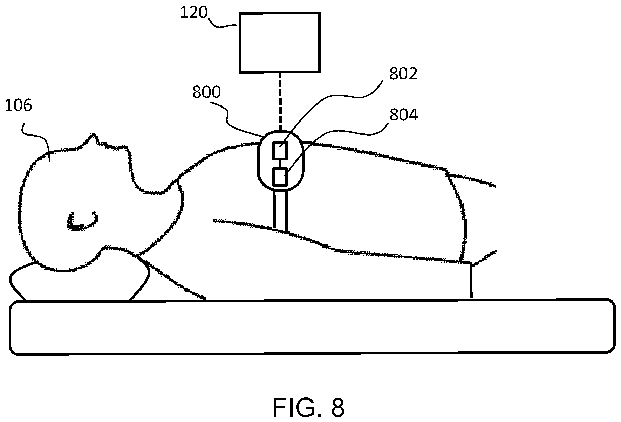

FIG. 8 depicts a third embodiment according to the invention. In the third embodiment, there is provided a therapy device 800 for providing sleep positional therapy. The therapy device 800 comprises a position sensor 802, a sensory stimulator 804 and the processing system 120. The processing system 120 has, for example, the same features are mentioned in the first embodiment or in the second embodiment, except for the following. The position sensor 802 is adapted to generate a position signal representative of whether the subject 106 is in a target position. The sensory stimulator 804 is adapted to provide sensory stimulation. The processing system 120 is configured to execute the computer program product of the preceding embodiments. The processing system 120 is configured to control the sensory stimulator 804 to provide stimulation based on the position signal, whether the first ratio exceeds the first threshold 401, and whether the REM occurrence value exceeds the second threshold 402.

For example, the processing system 120 is configured to control the sensory stimulator 604 to provide stimulation in case the subject 106 is in the target position and has a respiratory event, and to control the sensory stimulator 804 not to provide stimulation in case the subject 106 is in the target position and does not have a respiratory event.

In the first and second embodiments, the processing system 120 is described as a part of either the respiratory support device 100 or the therapy device 800. In an embodiment, the processing system 120 is a separate unit, that is connectable to, for example, the respiratory support device 100 or the therapy device 800. For example, the processing system 120 is provided in a mobile device, such as a mobile phone or tablet or a personal computer.

It will be understood that the disclosed method is a computer-implemented method. As such, there is also proposed a concept of a computer program comprising instructions for implementing the described method when the computer program is run on the processing system 120.

The skilled person would be readily capable of developing a processing system 120 for carrying out the method.

The processing system 120 performs the data processing to execute the method. The processing system 120 can be implemented in numerous ways, with software and/or hardware, to perform the various functions required. The processing system 120 typically employs one or more microprocessors that may be programmed using software (e.g. microcode) to perform the required functions. The processing system 120 may be implemented as a combination of dedicated hardware to perform some functions and one or more programmed microprocessors and associated circuitry to perform other functions.

Examples of circuitry that may be employed in the processing system 120 include, but are not limited to, conventional microprocessors, application specific integrated circuits (ASICs), and field-programmable gate arrays (FPGAs). Thus, the processing system 120 may be embodied as a digital and/or analog processing system 120.

The memory 202 may comprise any type of suitable storage media such as volatile and non-volatile computer memory 202 such as RAM, PROM, EPROM, and EEPROM. The memory 202 may be encoded with one or more programs that, when executed on the processing system 120, perform the required functions. Various storage media may be fixed within a processing system 120 or controller may be transportable, such that the one or more programs stored thereon can be loaded into a processing system 120.

Functions implemented by the processing system 120 may be implemented by a single unit or by multiple separate units which may together be considered to constitute the processing system 120. Such multiple units may be remote from each other and communicate with each other in a wired or wireless manner.

A computer program may be stored/distributed on the memory 202. The memory 202 comprises for example an optical storage medium or a solid-state medium supplied together with or as part of other hardware, but may also be distributed in other forms, such as via the Internet or other wired or wireless telecommunication systems.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1. A computer program product, comprising instructions which, when executed by a processing system (120), cause the processing system (120) to carry out a method for determining a sleep characteristic of a subject (106) during a sleep session, the method comprising:

receiving sleep stage information representative of sleep stages of the subject (106) during the sleep session;

receiving respiratory event information representative of respiratory events of the subject (106) during the sleep session;

determining a first ratio between a REM value and a NREM value,

wherein the REM value and the NREM value are based on the sleep stage information and the respiratory event information,

wherein the REM value is representative of a number of respiratory events during a REM time per unit of time,

wherein the REM time is representative of a time the subject (106) is asleep in a Rapid Eye Movement sleep stage during the sleep session;

wherein the NREM value is representative of a number of respiratory events during a NREM time per unit of time,

wherein the NREM time is representative of a time the subject (106) is asleep in a non-Rapid Eye Movement sleep stage during the sleep session;

determining whether the first ratio exceeds a first threshold (401);

determining a REM occurrence value representative of an amount of REM time during the sleep session; and

determining whether the REM occurrence value exceeds a second threshold (402, 403).

2. The computer program product according to claim 1,

wherein the REM value is representative of an apnea-hypopnea index (AHI) during the REM time,

wherein the NREM value is representative of an apnea-hypopnea index (AHI) during the NREM time,

wherein the first threshold (401) is representative of the apnea-hypopnea index (AHI) during the REM time being at least two times greater than the apnea-hypopnea index (AHI) during the NREM time.

3. The computer program product according to claim 1 or 2,

wherein the REM occurrence value is representative of a second ratio,

wherein the second ratio is between the REM time and a total sleep time of the sleep session.

4. The computer program product according to claim 3, wherein the second ratio is representative of the REM time being less than 25%, or less than 20%, or less than 15% of the total sleep time of the sleep session.

5. The computer program product according to any one of the preceding claims,

wherein the REM occurrence value is representative of a third ratio,

wherein the third ratio is between the number of respiratory events during the REM time and the number of respiratory events during the NREM time.

6. The computer program product according to claim 5, wherein the second threshold (403) is representative of the number of respiratory events during the REM time being 0.5 times the number of respiratory events during the NREM time.

7. The computer program product according to any one of the preceding claims,

wherein the first ratio is based on a probability difference representative of a difference between a probability of an occurrence of a respiratory event during the REM time and a probability of an occurrence of a respiratory event during the NREM time;

wherein the second ratio is based on the probability difference;

wherein the first threshold (401) is representative of a lower threshold value (611, 711) of the probability difference;

wherein the second threshold (402) is representative of an upper threshold value (612, 712) of the probability difference.

8. The computer program product according any one of the preceding claims, wherein the method comprises:

classifying the subject (106) as having REM-related obstructive sleep apnea (REM-OSA) based on the first ratio exceeding the first threshold (401) and based on the REM occurrence value exceeding the second threshold (402);

classifying the subject (106) as having both REM-related obstructive sleep apnea (REM-OSA) and REM-deprivation based on the first ratio exceeding the first threshold (401) and based on the REM occurrence value not exceeding the second threshold (402).

9. The computer program product according to claim 8, wherein the method comprises:
classifying the subject (106) as having non-REM-specific OSA based on the first ratio not exceeding the first threshold (401).

10. The computer program product according to any one of the preceding claims, wherein the method comprises providing an output signal (204) representative of an action based on whether the first ratio exceeds the first threshold (401) and based on whether the REM occurrence value exceeds the second threshold (402).

11. The computer program product according to claim 10, wherein the action comprises suggesting adjusting a sleep disordered breathing therapy performed on the subject (106) or start performing another type of sleep disordered breathing therapy on the subject (106).

12. A respiratory support device (100) for providing pressurized air to a subject (106), the respiratory support device (100) comprising:

a pressure source (108) adapted to generate the pressurized air;
a processing system (120) configured to execute the computer program product of any one of the preceding claims;
a sensor input (110) adapted to receive from a sensor system at least one sensor signal representative of the sleep stage information and the respiratory event information,
wherein the processing system (120) is configured to adjust a setting of the respiratory support device (100) based on whether the first ratio exceeds the first threshold (401) and based on whether the REM occurrence value exceeds the second threshold (402).

13. The respiratory support device (100) according to claim 12, wherein the processor system is configured to adjust the setting by reducing a pressure of the pressurized air.

14. The respiratory support device (100) according to claim 12 or 13, wherein the processing system (120) is configured to adjust the setting during the sleep session based on at least one of a REM time already spent during the sleep session and an expected REM time for a remainder of the sleep session.

15. A therapy device (800) for providing sleep positional therapy, comprising:

a position sensor (802) adapted to generate a position signal representative of whether the subject (106) is in a target position;
a sensory stimulator (804) adapted to provide sensory;
a processing system (120) configured to execute the computer program product of any claims 1-11,
wherein the processing system (120) is configured to control the sensory stimulator (604) to provide stimulation based on:

the position signal,
whether the first ratio exceeds the first threshold (401), and
whether the REM occurrence value exceeds the second threshold (402).

FIG. 1

FIG. 2

FIG. 3

FIG. 4

| | |
|---|---|
| Receiving sleep stage information | 501 |

↓

| | |
|---|---|
| Receiving respiratory event information | 502 |

↓

| | |
|---|---|
| Determining a first ratio between a REM value and a NREM value | 503 |

↓

| | |
|---|---|
| Determining a REM occurrence value | 504 |

↓

| | |
|---|---|
| Does the first ratio exceed the first threshold? | 505 |

Yes         No

506   Determining whether the REM occurrence value exceeds a second threshold      Classifying the subject as having non-REM-specific OSA   507

No

Yes    Classifying the subject as having both REM-related obstructive sleep apnea (REM-OSA) and REM deprivation   509

508   Classifying the subject as having REM-related obstructive sleep apnea

**FIG. 5**

FIG. 6

FIG. 7

FIG. 8

Europäisches
Patentamt

European
Patent Office

Office européen
des brevets

## EUROPEAN SEARCH REPORT

**Application Number**

EP 24 16 8642

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 117 504 074 A (FEIYINUO TECH CO LTD) 6 February 2024 (2024-02-06) | 1-14 | INV. A61B5/00 A61B5/087 A61M16/00 |
| A | * page 7, paragraph 24 - paragraph 25 * * page 9, paragraph 47 - paragraph 48 * * page 10, paragraph 57 * * page 13, paragraph 98 * * page 15, paragraph 125 - paragraph 131 * ----- | 15 | |
| A | ABDUL REHMAN RISHI: "Rapid eye movement related obstructive sleep apnea: Where do we stand?", RESPIRATORY INVESTIGATION, [Online] vol. 59, no. 5, 7 July 2021 (2021-07-07), pages 589-595, XP0093156186, ISSN: 2212-5345, DOI: 10.1016/j.resinv.2021.06.006 Retrieved from the Internet: URL:https://dx.doi.org/10.1016/j.resinv.20 21.06.006> [retrieved on 2024-04-30] * table 1 * ----- | 1-15 | |
| A | BABAK MOKHLESI: ""REM-related" Obstructive Sleep Apnea: An Epiphenomenon or a Clinically Important Entity?", SLEEP, [Online] vol. 35, no. 1, 1 January 2012 (2012-01-01), pages 5-7, XP093158302, US ISSN: 0161-8105, DOI: 10.5665/sleep.1570 Retrieved from the Internet: URL:https://www.sciencedirect.com/science/ article/pii/S2212534521001155/pdfft?md5=dd a3068b10a5e082144fc9f82b6cd340&pid=1-s2.0- S2212534521001155-main.pdf> [retrieved on 2024-04-29] * page 5 * ----- -/-- | 1-15 | TECHNICAL FIELDS SEARCHED (IPC) A61M A61B |

The present search report has been drawn up for all claims

| Place of search | Date of completion of the search | Examiner |
|---|---|---|
| The Hague | 29 May 2024 | Costa Angeli, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 4

Europäisches
Patentamt

European
Patent Office

Office européen
des brevets

# EUROPEAN SEARCH REPORT

**Application Number**

EP 24 16 8642

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/218293 A1 (CHOU CHANG-AN [TW]) 14 July 2022 (2022-07-14) | 15 | |
| A | * paragraph [0060] * <br> * paragraph [0110] - paragraph [0111] * <br> * paragraph [0134] * <br> * figure 5 * | 1-14 | |
| A | RISSANEN M ET AL: "Total durations of respiratory events are modulated within REM and NREM sleep by sleeping position and obesity in OSA patients", SLEEP MEDICINE, ELSEVIER, AMSTERDAM, NL, vol. 81, 17 February 2021 (2021-02-17), pages 394-400, XP086553094, ISSN: 1389-9457, DOI: 10.1016/J.SLEEP.2021.02.020 [retrieved on 2021-02-17] * page 2 * | 1-15 | |
| A | US 2023/173221 A1 (SHOULDICE REDMOND [IE]) 8 June 2023 (2023-06-08) * page 4 * | 1-15 | TECHNICAL FIELDS SEARCHED (IPC) |
| A | BRIAN B KOO ET AL: "REM rebound and CPAP compliance", SLEEP MEDICINE, ELSEVIER, AMSTERDAM, NL, vol. 13, no. 7, 5 March 2012 (2012-03-05), pages 864-868, XP028426336, ISSN: 1389-9457, DOI: 10.1016/J.SLEEP.2012.03.019 [retrieved on 2012-04-28] * page 1 * | 1-15 | |

-/--

The present search report has been drawn up for all claims

| Place of search | Date of completion of the search | Examiner |
|---|---|---|
| The Hague | 29 May 2024 | Costa Angeli, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 24 16 8642

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Anonymous: "Sleep Physiology - Sleep Disorders and Sleep Deprivation - NCBI Bookshelf", , 1 January 2006 (2006-01-01), XP093156994, Retrieved from the Internet: URL:https://www.ncbi.nlm.nih.gov/books/NBK19956/ [retrieved on 2024-04-30] * paragraph [0006] * * paragraph [0160] - paragraph [0168] * * figure 3 * | 1-15 | |
| A | BABAK MOKHLESI: "Obstructive Sleep Apnea during REM Sleep and Hypertension. Results of the Wisconsin Sleep Cohort", AMERICAN JOURNAL OF RESPIRATORY AND CRITICAL CARE MEDICINE, vol. 190, no. 10, 15 November 2014 (2014-11-15), pages 1158-1167, XP093158291, US ISSN: 1073-449X, DOI: 10.1164/rccm.201406-1136OC * figure 1 * | 1-15 | |

TECHNICAL FIELDS
SEARCHED     (IPC)

-/--

The present search report has been drawn up for all claims

| Place of search | Date of completion of the search | Examiner |
|---|---|---|
| The Hague | 29 May 2024 | Costa Angeli, M |

EPO FORM 1503 03.82 (P04C01)

Europäisches
Patentamt

European
Patent Office

Office européen
des brevets

# EUROPEAN SEARCH REPORT

**Application Number**

EP 24 16 8642

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Mathias J Holmberg: "Estimating Risk Ratios and Risk Differences: Alternatives to Odds Ratios", JAMA : the journal of the American Medical Association, 15 September 2020 (2020-09-15), pages 1098-1099, XP093157531, Chicago DOI: 10.1001/jama.2020.12698 Retrieved from the Internet: URL:https://www.feinberg.northwestern.edu/sites/firstdailylife/docs/JAMA_Estimating_Risk_Ratios_and_Risk_Differences_Alternatives_to_Odds_Ratios.pdf [retrieved on 2024-04-29] * Section:What are risk ratio and risk difference; page 1 * | 1-15 | |
| A | HOSHINO TETSURO ET AL: "Estimated respiratory arousal threshold in patients with rapid eye movement obstructive sleep apnea", SLEEP AND BREATHING - SCHLAF UND ATMUNG, DRUCKBILD, TITISEE-NEUSTADT, DE, vol. 26, no. 1, 17 May 2021 (2021-05-17), pages 347-353, XP037694231, ISSN: 1520-9512, DOI: 10.1007/S11325-021-02399-9 [retrieved on 2021-05-17] * page 2 * * figure 1 * | 1-15 | TECHNICAL FIELDS SEARCHED (IPC) |

The present search report has been drawn up for all claims

| Place of search | Date of completion of the search | Examiner |
|---|---|---|
| The Hague | 29 May 2024 | Costa Angeli, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 16 8642

This annex lists the patent family members relating to the patent documents cited in  the above-mentioned European search report.
The members are as contained in the European Patent Office EDP file on
The European Patent Office is in no way liable for these particulars which are merely given for the purpose of information.

29-05-2024

| Patent document cited in search report | | Publication date | Patent family member(s) | | Publication date |
|---|---|---|---|---|---|
| CN 117504074 | A | 06-02-2024 | NONE | | |
| US 2022218293 | A1 | 14-07-2022 | JP | 3243566 U | 04-09-2023 |
| | | | JP | 2022532849 A | 20-07-2022 |
| | | | US | 2022218293 A1 | 14-07-2022 |
| | | | WO | 2020228725 A1 | 19-11-2020 |
| US 2023173221 | A1 | 08-06-2023 | AU | 2021265344 A1 | 08-12-2022 |
| | | | CN | 115836358 A | 21-03-2023 |
| | | | EP | 4143849 A1 | 08-03-2023 |
| | | | JP | 2024512835 A | 21-03-2024 |
| | | | US | 2023173221 A1 | 08-06-2023 |
| | | | WO | 2021220247 A1 | 04-11-2021 |

For more details about this annex : see Official Journal of the European Patent Office, No. 12/82